# EUROPÄISCHE PATENTANMELDUNG

(11) **EP 2 465 459 A1**
(43) Veröffentlichungstag der Anmeldung: **20.06.2012**
(21) Anmeldenummer: 10195058.2
(22) Anmeldetag: 15.12.2010
(51) Int. Cl.: A61B 19/00, A61C 19/00, A61B 19/02

(54) **Reinigungs- oder Pflegekassette für medizinische, insbesondere zahnärztliche, Instrumente**

(71) Anmelder: W & H Dentalwerk Bürmoos GmbH, 5111 Bürmoos (AT)
(72) Erfinder: Meburger, Jürgen, 5112, Lamprechtshausen (AT)

(57) **Zusammenfassung**

Reinigungs- oder Pflegekassette (1) zur Aufnahme von medizinischen, insbesondere zahnärztlichen, Instrumenten, aufweisend ein Gehäuse mit zumindest einem ersten und zweiten Gehäuseteil (2, 3), welche jeweils zumindest eine Deckel- oder Bodenfläche (4, 5) sowie zumindest eine Seitenfläche (6, 7) aufweisen, und zumindest eine zwischen den zwei Gehäuseteilen (2, 3) angeordnete Anschlussvorrichtung (8) für zumindest ein medizinisches, insbesondere zahnärztliches, Instrument, wobei das erste und zweite Gehäuseteil (2, 3) drehbar über eine Drehachse (9) miteinander verbunden sind und die Drehachse (9) gleich beabstandet zu den Deckel- oder Bodenflächen (4, 5) beider Gehäuseteile (2, 3) angeordnet ist.

## Beschreibung

Die vorliegende Erfindung bezieht sich auf eine Reinigungs- oder Pflegekassette für medizinische, insbesondere zahnärztliche, Instrumente nach dem Oberbegriff des Anspruchs 1.

Derartige Reinigungs- oder Pflegekassetten dienen zur Aufnahme von zu reinigenden und/ oder pflegenden medizinischen, insbesondere zahnärztlichen, Instrumenten in Reinigungs- oder Pflegegeräten.

Als zu reinigende Instrumente werden insbesondere gerade, gebogene, oder pistolenförmige Handstücke als auch Teile von Handstücken, z. B. Handstückköpfe mit einer Werkzeugaufnahme zur Aufnahme eines Behandlungswerkzeugs, Adapter und Kupplungen verstanden. Durch die Handstücke erstrecken sich oftmals Versorgungsleitungen zum Antrieb eines Behandlungswerkzeugs sowie Fluidleitungen. Bei den Leitungen handelt es sich insbesondere um Getriebekanäle oder Fluidkanäle für Luft, Wasser oder Spray.

Nach dem Betrieb der oben angeführten Instrumente bedarf es in regelmäßigen Abständen einer Reinigung und Pflege der Leitungen, insbesondere der Antriebskanäle sowie der Fluidkanäle. Hierzu stehen dem Anwender eine Vielzahl an Reinigung- und Pflegegeräte zu Verfügung.

Unter Reinigungs- oder Pflegegerät sind Vorrichtungen zu verstehen, welche die medizinischen, insbesondere zahnärztlichen, Instrumente reinigen, insbesondere von Verschmutzungen befreien, desinfizieren, sterilisieren und/ oder pflegen. Zur Reinigung und/ oder Pflege der medizinischen, insbesondere zahnärztlichen, Instrumente können Fluide, insbesondere Flüssigkeiten, wie zum Beispiel Kaltwasser oder Heißwasser, oder Gase, wie zum Beispiel Wasserdampf oder Druckluft, Desinfektionsmittel, Sterilisationsmittel, oder Schmiermittel, wie zum Beispiel natürliche oder synthetische Schmieröle, verwendet werden. Diese Geräte weisen, neben einer Vorrichtung zur Außenreinigung, insbesondere eine Schnittstelle zur Reinigung der im Inneren des Handstücks angeordneten Versorgungs- und Fluidleitungen auf.

Die medizinischen, insbesondere zahnärztlichen, Instrumente werden vorzugsweise mittels einer Reinigungs- oder Pflegekassette in der Reinigungs- oder Pflegekammer des Reinigungs- oder Pflegegeräts aufgenommen und insbesondere zu der Schnittstelle des Reinigungs- oder Pflegegeräts zur Reinigung und Pflege der Versorgungs- und Fluidleitungen der medizinischen, insbesondere zahnärztlichen, Instrumente positioniert.

Eine derartige Reinigungs- oder Pflegekassette ist aus der EP1779800 B1 bekannt.

Diese bekannte Reinigungs- oder Pflegekassette weist ein Gehäuse mit einem oberen Gehäuseteil, insbesondere einem Deckel, und einem zweiten Gehäuseteil, insbesondere einem Basisteil, auf. Zwischen beiden Gehäuseteilen ist eine Anschlussvorrichtung zur Aufnahme zumindest eines medizinischen, insbesondere zahnärztlichen, Instruments um eine Querachse drehbar gelagert. Da das Verbinden des medizinischen, insbesondere zahnärztlichen, Instruments mit der Anschlussvorrichtung in einer etwa vertikalen Pflegestellung für den Anwender umständlich ist, ist die Anschlussvorrichtung um eine horizontale Querachse zwischen einer Reinigungsstellung in eine in etwa 45 Grad zur vertikalen Achse der Reinigungs- oder Pflegekassette angeordneten Bedienungsstellung am unteren Gehäuseteil schwenkbar gelagert. Zum Verschließen und Öffnen der Reinigungskammer ist der Deckel am oberen Rand des Basisteils um eine Drehachse schwenkbar gelagert.

Als nachteilig dieser Ausgestaltung der Reinigungs- oder Pflegekassette erweist sich die Bedienung, insbesondere das Beladen, der Reinigungskammer mit den medizinischen, insbesondere zahnärztlichen Instrumenten. Um die Kassette, insbesondere die Anschlussvorrichtung der Reinigungs- oder Pflegekassette, für den Anwender in gewünschter Weise, insbesondere in der Bedienungsstellung, zugänglich zu machen, muss der Anwender die Reinigungs- oder Pflegekassette richtig herum positionieren. Er muss darauf achten, dass beim Öffnen der Kassette die Oberseite, nämlich der Deckel, oberhalb des Basisteils angeordnet ist.

Einen weiteren Nachteil der im Stand der Technik bekannten Ausführungsform stellt die aufwendige Fertigung der Reinigungs- oder Pflegekassette dar. Durch die unterschiedliche Ausgestaltung der beiden Gehäuseteile ist es notwendig für beide Bauteile ein eigenes Fertigungswerkzeug bzw. eine eigene Fertigungsmaschine herzustellen bzw. bereitzustellen.

Der vorliegenden Erfindung liegt daher die Aufgabe zugrunde eine Reinigungs- oder Pflegekassette zu schaffen, welche es ermöglicht die medizinischen, insbesondere zahnärztliche, Instrumente bedienungsfreundlich in der Reinigungs- oder Pflegekassette aufzunehmen. Des Weiteren soll eine einfache Herstellung der Reinigungs- oder Pflegekassette gewährleistet werden.

Gemäß einem Ausführungsbeispiel weist die Reinigungs- oder Pflegekassette zur Aufnahme von medizinischen, insbesondere zahnärztlichen, Instrumenten ein Gehäuse mit zumindest einem ersten und zweiten Gehäuseteil, welche jeweils zumindest eine Deckel- oder Bodenfläche sowie zumindest eine Seitenfläche aufweisen, und zumindest eine zwischen den zwei Gehäuseteilen angeordnete Anschlussvorrichtung für zumindest ein medizinisches, insbesondere zahnärztliches, Instrument auf, wobei das erste und zweite Gehäuseteil drehbar über eine Drehachse miteinander verbunden sind und die Drehachse gleich beabstandet zu den Deckel- oder Bodenflächen beider Gehäuseteile angeordnet ist.

Unter dem Begriff Reinigungs- oder Pflegekassette sind insbesondere Vorrichtungen zu verstehen, welche zur Aufnahme von medizinischen, insbesondere zahnärztlichen, Instrumenten, in Reinigungs- oder Pflegegeräten zur Reinigung, Pflege, Desinfektion und/ oder Sterilisation dienen. Des Weiteren können derartige Kassetten verwendet werden, um die medizinischen Instrumente in gereinigtem oder sterilem Zustand aufzubewahren.

Gemäß einem Ausführungsbeispiel bildet die Anschlussvorrichtung für das zumindest eine medizinische, insbesondere zahnärztliche, Instrument die Drehachse der Reinigungs- oder Pflegekassette. Die Anschlussvorrichtung weist hierzu einen Träger mit vorzugsweise zwei Lagerzapfen zur Lagerung zumindest eines der beiden Gehäuseteile auf, um welche das Gehäuseteil drehbar ist. An dem Träger ist zumindest ein Anschlussstück angeordnet, um zumindest ein medizinisches, insbesondere zahnärztliches, Instrument aufzunehmen. Des Weiteren ist es möglich das medizinische, insbesondere zahnärztliche Instrument, über einen Anschlusszapfen mit dem Anschlussstück zu verbinden.

Das Anschlussstück der Anschlussvorrichtung ist vorzugsweise relativ zu dem Träger drehbar gelagert, so dass das mit dem Anschlussstück verbundene medizinische, insbesondere zahnärztliche, Instrument relativ zu dem Träger und insbesondere zu der Reinigungs- oder Pflegekassette drehbar ist. Hierdurch ist es möglich die aufgenommen medizinischen Instrumente, insbesondere zahnärztliche Hand- und Winkelstücke, platzsparend in der Reinigungs- oder Pflegekassette aufzunehmen. Das Anschlussstück selbst erstreckt sich vorzugsweise bis an die Rückseite des Trägers der Anschlussvorrichtung oder ist zumindest von der Rückseite durch eine Öffnung in dem Träger zugänglich.

Die Anschlussvorrichtung bildet ein weiteres Gehäuseteil der Reinigungs- oder Pflegekassette. Hierdurch ist es möglich eine Medienzuführung eines Reinigungs- oder Pflegegeräts mit der Reinigungs- oder Pflegekassette, insbesondere direkt mit der Anschlussvorrichtung, zu koppeln. Dafür weist das zumindest eine Anschlussstück der Anschlussvorrichtung einen ersten Kupplungsbereich für ein medizinisches Instrument sowie einen zweiten Kupplungsbereich zur Verbindung mit einer Medienzuführung eines Reinigungs- oder Pflegegeräts auf. Des Weiteren beinhaltet das zumindest eine Anschlussstück zumindest einen, vorzugsweise drei, Medienkanäle, um den Versorgungs- und Fluidleitungen der angekuppelten medizinischen Instrumente Reinigungs- oder Pflegemittel des Reinigungs- oder Pflegegeräts zuzuführen.

Die Anschlussvorrichtung selbst ist insbesondere um eine Querachse zwischen einer Reinigungs- oder Pflegestellung und einer Bedienungsstellung drehbar gelagert. Bevorzugt ist hierbei die Querachse gleich beabstandet zu den Seiten der Anschlussvorrichtung angeordnet, welche den Deckel- oder Bodenflächen des ersten und zweiten Gehäuseteils zugewandt sind, so dass die Anschlussvorrichtung gleichmäßig in beide Richtungen um die Querachse schwenkbar gelagert ist.

Gemäß einem weiteren Ausführungsbeispiel schneidet die Mittelachse des zumindest einen Anschlussstücks der Anschlussvorrichtung die Querachse und/ oder die Drehachse der Anschlussvorrichtung, so dass das Anschlussstück mittig an der Anschlussvorrichtung positioniert ist. Hierbei liegt vorzugsweise die Querachse der Anschlussvorrichtung in der Drehachse der zumindest zwei Gehäuseteile der Reinigungs- oder Pflegekassette. Des Weiteren schneidet im geschlossenen Zustand der Reinigungs- oder Pflegekassette vorzugsweise die Kontaktfläche des ersten und zweiten Gehäuseteils, in der die zumindest eine Seitenfläche des ersten Gehäuseteils die zumindest eine Seitenfläche des zweiten Gehäuseteils kontaktiert, die Drehachse der Reinigungs- oder Pflegekassette.

Das erste und zweite Gehäuseteil weisen jeweils zumindest ein Verriegelungselement auf, so dass die zumindest zwei Gehäuseteile der Reinigungs- oder Pflegekassette im geschlossenen Zustand miteinander lösbar verriegelbar sind. Des Weiteren ist vorzugsweise an beiden Gehäuseteilen zumindest eine Öffnung zur Aufnahme zumindest einer Reinigungsdüse eines Reinigungs- oder Pflegegeräts in der Reinigungs- oder Pflegekassette angeordnet, wobei die zumindest eine Öffnung bevorzugt an der der Anschlussvorrichtung gegenüberliegenden Seite der Reinigungs- oder Pflegekassette angeordnet ist.

Um bei Anordnung der Reinigungs- oder Pflegekassette in einem Reinigungs- oder Pflegegerät ein Reinigungs- oder Pflegemittel an der Außenseite der Reinigungs- oder Pflegekassette vorbeileiten zu können, weist das erste und zweite Gehäuseteil an der Außenseite der Deckel- oder Bodenfläche jeweils zumindest einen Gehäusevorsprung auf. Des Weiteren ist an der Außenseite der Deckel- oder Bodenflächen vorzugsweise zumindest ein Gehäuserücksprung angeordnet, so dass ein Gehäusevorsprung einer ersten Reinigungs- oder Pflegekassette in einen Gehäuserücksprung einer zweiten Reinigungs- oder Pflegekassette eingreift. Hierdurch ist es möglich mehrere Reinigungs-. oder Pflegekassetten sicher übereinander zu stapeln.

Das erste und zweite Gehäuseteil weist jeweils zumindest zwei Lagerstellen zur Aufnahme der Drehachse der Reinigungs- oder Pflegekassette auf. In montiertem Zustand ist eine erste Lagerstelle des ersten Gehäuseteils zwischen den zwei Lagerstellen des zweiten Gehäuseteils und die zweite Lagerstelle außerhalb angeordnet.

Gemäß einem bevorzugten Ausführungsbeispiel ist das Gehäuse, insbesondere das erste und zweite Gehäuseteil, der Reinigungs- oder Pflegekassette im montierten Zustand drehsymmetrisch ausgebildet. Durch die Drehung des ersten Gehäuseteils um einen Winkel von 180 Grad um die Drehsymmetrieachse der Reinigungs- oder Pflegekassette, wird dieses auf dem zweiten Gehäuseteil abgebildet. Auf Grund dieser Ausgestaltung beider Gehäuseteile der Kassette ist es möglich das erste und zweite Gehäuseteil durch ein identisches Bauteil auszugestalten.

Gemäß einem weiteren bevorzugten Ausführungsbeispiel ist des Weiteren die Anschlussvorrichtung der Reinigung- oder Pflegekassette, insbesondere deren Träger, drehsymmetrisch zu der Drehsymmetrieachse der Reinigungs- oder Pflegekassette ausgebildet. Durch die Drehung der Anschlussvorrichtung, insbesondere des Trägers, um einen Winkel von 180 Grad um die Drehsymmetrieachse der Reinigungs- oder Pflegekassette wird die Anschlussvorrichtung bzw. der Träger wieder auf sich selbst abgebildet.

Die vorliegende Erfindung zeichnet sich durch eine Reihe erheblicher Vorteile aus.

Durch die Anordnung der Drehachse der Reinigungs- oder Pflegekassette gleich beabstandet zu den Deckel- oder Bodenflächen des ersten und zweiten Gehäuseteils, wird es ermöglicht den Reinigungs- oder Pflegeraum der Reinigungs- oder Pflegekassette durch Öffnen des ersten und/ oder zweiten Gehäuseteils in gleicher Weise zugänglich zu machen.

Des Weiteren wird durch die drehsymmetrische Ausbildung des Gehäuses, insbesondere des ersten und zweiten Gehäuseteils, sowie insbesondere der Anschlussvorrichtung der Reinigungs- oder Pflegekassette, eine einfache Bedienung, insbesondere Bestückung der Anschlussvorrichtung mit den medizinischen, insbesondere zahnärztlichen, Instrumenten, gewährleistet. Eine selektive Positionierung der Reinigungs- oder Pflegekassette ist somit nicht mehr notwendig. Mittels Öffnen des ersten und/ oder zweiten Gehäuseteils ist die Anschlussvorrichtung für den Anwender in der Bedienungsstellung zugänglich.

Einen weiteren Vorteil der Erfindung stellt die kostengünstige Fertigung des Gehäuses der Reinigungskassette dar. Durch die Ausgestaltung beider Gehäuseteile durch zwei Bauteile mit identischer Form, ist es möglich das zweiteilige Gehäuse mittels einem einzigen Fertigungswerkzeug, bzw. einer einzigen Fertigungsmaschine herzustellen.

Im Rahmen der Erfindung versteht es sich selbstverständlich, dass die Reinigungs- oder Pflegekassette nicht auf das oben beschriebene Ausführungsbeispiel beschränkt ist.

Nachfolgend wird die Erfindung anhand eines Ausführungsbeispiels und in Verbindung mit den beigefügten Zeichnungen erläutert.

Dabei zeigt:
Figur 1 ein Ausführungsbeispiel der Reinigungs- oder Pflegekassette in der Außenansicht,
Figur 2 eine Seitenansicht der Reinigungs- oder Pflegekassette aus Figur 1,
Figur 3 ein Ausführungsbeispiel des ersten oder zweiten Gehäuseteils der Reinigungs-oder Pflegekassette,
Figur 4 die Anschlussvorrichtung der Reinigungs- oder Pflegekassette in der Bedienungsstellung;
Figur 5 eine Seitenansicht der Anschlussvorrichtung und des ersten oder zweiten Gehäuseteils der Reinigungs- oder Pflegekassette in der Bedienungsstellung,
Figur 6 ein Ausführungsbeispiel der Anschlussvorrichtung der Reinigungs- oder Pflegekassette;

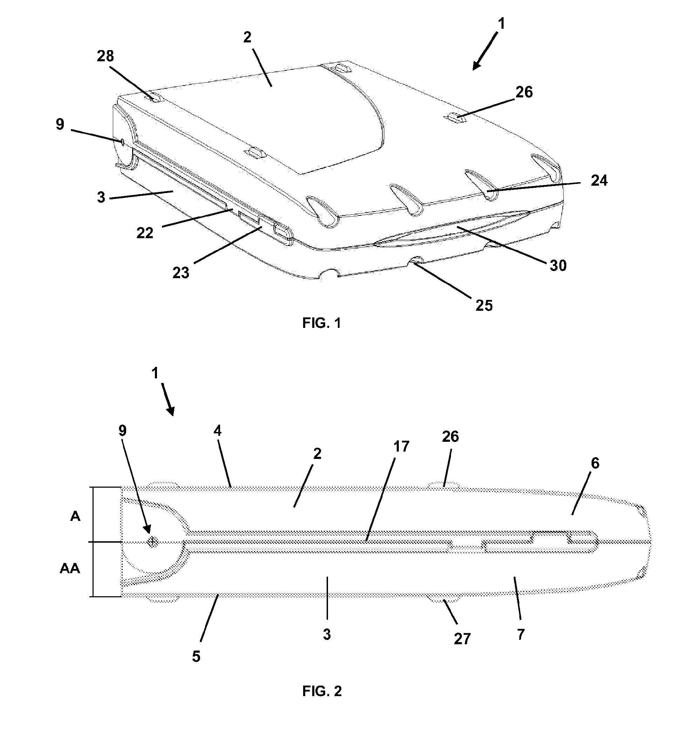

In Figur 1 ist ein Ausführungsbeispiel der Reinigungs- oder Pflegekassette 1 in der Außenansicht abgebildet. Hierbei wird insbesondere das Gehäuse mit einem ersten Gehäuseteil 2 sowie einem zweiten Gehäuseteil 3 gezeigt. Beide Gehäuseteile 2, 3 sind über eine Drehachse 9 drehbar miteinander verbunden. Sowohl das erste als auch das zweite Gehäuseteile 2, 3 weisen zumindest ein Verriegelungselement 22, 23 auf, so dass die zumindest zwei Gehäuseteile 2, 3 im geschlossenen Zustand miteinander lösbar verriegelbar sind. Des Weiteren weisen beide Gehäuseteil 2, 3 jeweils zumindest eine Öffnung 24, 25 zur Aufnahme zumindest einer Reinigungsdüse eines Reinigungs- oder Pflegegeräts in der Reinigungs- oder Pflegekassette 1 auf. Eine weitere Gehäuseöffnung 30, welche insbesondere durch das erste und zweite Gehäuseteil 2, 3 im geschlossenen Zustand gebildet wird, ermöglicht dem Anwender das Hintergreifen zumindest eines der beiden Gehäuseteile 2, 3, um die Reinigungs- oder Pflegekassette 1 zu öffnen. An den Außenseiten beider Gehäuseteile 2, 3 sind jeweils zumindest ein Gehäusevorsprung 26 sowie ein Gehäuserücksprung 28 angeordnet. Mittels des Gehäusevorsprungs 26 wird gewährleistet, dass bei Anordnung der Reinigungs- oder Pflegekassette 1 in einem Reinigungs- oder Pflegegerät ein Reinigungs- oder Pflegemittel an der Außenseite der Reinigungs- oder Pflegekassette 1 vorbeileitbar ist. Der Gehäusevorsprung 26 dient somit als Abstandshalter zwischen dem Gehäuse der Reinigungs- oder Pflegekassette 1 und der Innenwand des Reinigungs- oder Pflegegeräts. Der zumindest eine Gehäuserücksprung 28 an beiden Gehäuseteilen 2, 3 dient zum Stapeln mehrere Kassetten 1 übereinander. Hierbei greift ein Gehäusevorsprung 26, 27 einer ersten Reinigungs- oder Pflegekassette 1 in einen Gehäuserücksprung 28 einer zweiten Reinigungs- oder Pflegekassette 1.

Figur 2 zeigt die Reinigungs- oder Pflegekassette 1 aus Figur 1 in der Seitenansicht. Beide Gehäuseteile 2, 3 bestehen zumindest aus einer Deckel- oder Bodenfläche 4, 5 sowie aus zumindest einer Seitenfläche 6, 7. Des Weiteren weisen die Deckel- oder Bodenflächen 4, 5 vorzugsweise zumindest einen Gehäusevorsprung 26 sowie zumindest einen Gehäusevorsprung 27 auf. Die Drehachse 9, über welche beide Gehäuseteile 2, 3 drehbar miteinander verbunden sind, ist gleich beabstandet zu den Deckel- oder Bodenflächen 4, 5 beider Gehäuseteile 2, 3. Der Abstand A der Drehachse 9 zu der Deckel- oder Bodenfläche 4 des ersten Gehäuseteils 2 entspricht dem Abstand AA der Drehachse 9 zu der Deckel- oder Bodenfläche 5 des zweiten Gehäuseteils 3. Im geschlossenen Zustand der Reinigungs- oder Pflegekassette 1 schneidet somit die Kontaktfläche 17 des ersten und zweiten Gehäuseteils 2, 3, an der die zumindest eine Seitenfläche 6 des ersten Gehäuseteils 2 die zumindest eine Seitenfläche 7 des zweiten Gehäuseteils 3 kontaktiert, die Drehachse 9 der Reinigungs- oder Pflegekassette 1.

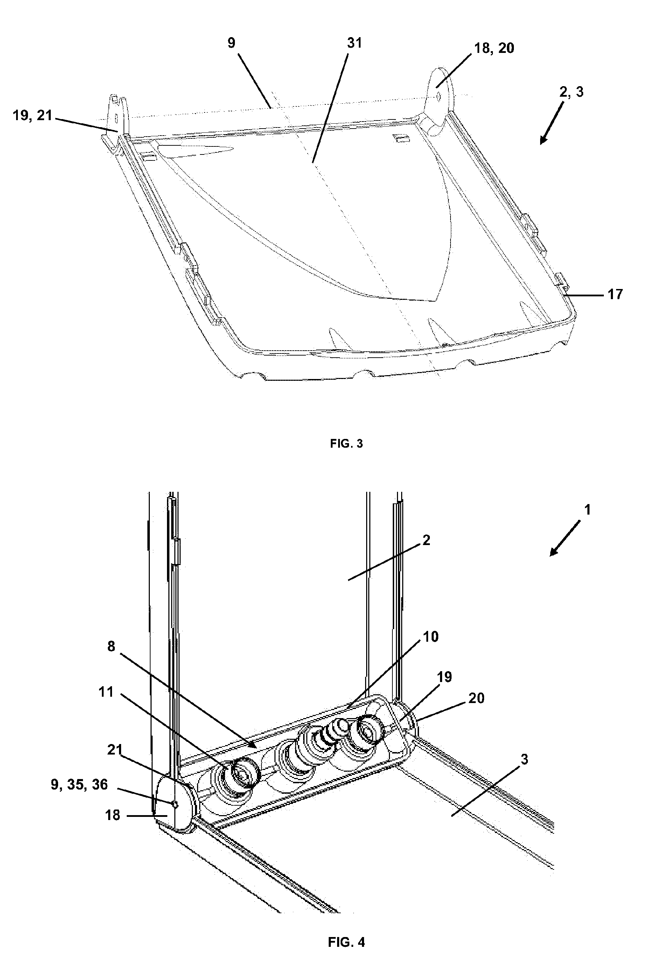

Das aus dem ersten und zweiten Gehäuseteil 2, 3 bestehende Gehäuse der Reinigungs-oder Pflegekassette 1 ist bevorzugt drehsymmetrisch ausgebildet. Durch die Drehung des ersten Gehäuseteils 2 um einen Winkel von 180 Grad um die Drehsymmetrieachse 31, siehe Figur 3, der Reinigungs- oder Pflegekassette 1, wird dieses auf dem zweiten Gehäuseteil 3 abgebildet. Durch diese Ausgestaltung beider Gehäuseteile 2, 3 der Kassette 1 ist es möglich das erste und zweite Gehäuseteil 2, 3 durch eine identische Bauform auszubilden. In Figur 3 ist ein Ausführungsbeispiel des ersten oder zweiten Gehäuseteils 2, 3 der Reinigungs- oder Pflegekassette 1 gezeigt. Das erste und zweite Gehäuseteil 2, 3 weist jeweils zumindest zwei Lagerstellen 18, 19; 20, 21 zur Aufnahme der Drehachse 9 der Reinigungs- oder Pflegekassette 1 auf. Beide Lagerstellen 18, 19 oder 20, 21 des ersten oder zweiten Gehäuseteils 2, 3 sind hierzu unterschiedlich beabstandet zu der Drehsymmetrieachse 31 der Reinigungs- oder Pflegekassette, so dass in montiertem Zustand eine erste Lagerstelle 19 des ersten Gehäuseteils 2 zwischen den zwei Lagerstellen 20, 21 des zweiten Gehäuseteils 3 und die zweite Lagerstelle 18 außerhalb angeordnet ist. Des Weiteren weisen beide Gehäuseteile jeweils zumindest eine Kontaktfläche 17 auf, an der die zumindest eine Seitenfläche 6 des ersten Gehäuseteils 2 die zumindest eine Seitenfläche 7 des zweiten Gehäuseteils 3 kontaktiert.

Figur 4 zeigt die Anschlussvorrichtung 8 der Reinigungs- oder Pflegekassette 1 in der Bedienungsstellung. Die zwischen den zwei Gehäuseteilen 2, 3 angeordnete Anschlussvorrichtung 8 ist über zumindest zwei Lagerzapfen 35, 36 in den Lagerstellen 18, 19; 20, 21 des ersten und zweiten Gehäuseteils 2, 3 drehbar gelagert. Bevorzugt bildet die Anschlussvorrichtung 8 für das zumindest eine medizinische, insbesondere zahnärztliche, Instrument die Drehachse 9 der Reinigungs- oder Pflegekassette 1, um welche zumindest ein Gehäuseteil 2, 3 drehbar ist. Die Anschlussvorrichtung 8 selbst besteht insbesondere aus einem Träger 10 sowie zumindest aus einem Anschlussstück 11 für ein medizinisches, insbesondere zahnärztliches, Instrument. Das Anschlussstück 11 der Anschlussvorrichtung 8 ist relativ zu dem Träger 10 drehbar gelagert, so dass das mit dem Anschlussstück 11 verbundene medizinische, insbesondere zahnärztliche, Instrument relativ zu dem Träger 10 drehbar ist und platzsparend in der Reinigungs- oder Pflegekassette 1 aufgenommen werden kann.

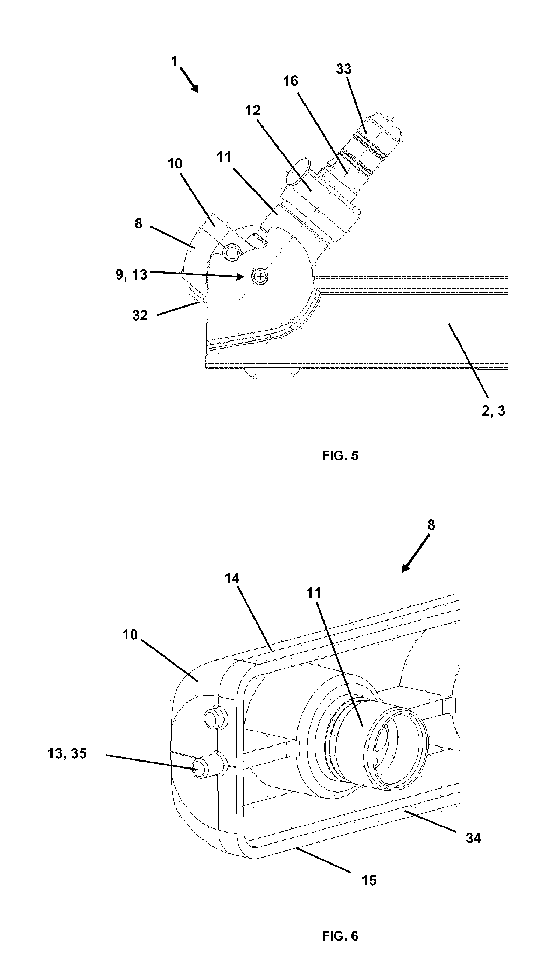

In Figur 5 ist die Anschlussvorrichtung 8 und das erste oder zweite Gehäuseteil 2, 3 der Reinigungs- oder Pflegekassette 1 in der Seitenansicht gezeigt. Die Anschlussvorrichtung 8 selbst ist drehbar um eine Querachse 13 gelagert. In diesem bevorzugten Ausführungsbeispiel liegt die Querachse 13 der Anschlussvorrichtung 8 in der Drehachse 9 der zumindest zwei Gehäuseteile 2, 3 der Reinigungs- oder Pflegekassette 1 oder ist mit dieser identisch. Die Mittelachse 16 des zumindest einen Anschlussstücks 11 der Anschlussvorrichtung 8 schneidet vorzugsweise die Querachse 13 der Anschlussvorrichtung und/ oder die Drehachse 9 der zumindest zwei Gehäuseteile 2, 3, wodurch das zumindest eine Anschlussstück 11 mittig an der Anschlussvorrichtung 8 positionierbar ist. Durch die Anordnung des zumindest einen Anschlussstücks 11 mittig an der Anschlussvorrichtung 8, wird somit gewährleistet, dass das über das zumindest eine Anschlussstück 11 oder über den mit dem Anschlussstück 11 gekuppelten Anschlusszapfen 12, insbesondere über dessen Kupplungsbereich 33, aufgenommene medizinische Instrument, gleich zugänglich über das erste und/ oder zweite Gehäuseteil 2, 3, auf der Anschlussvorrichtung 8, insbesondere auf dessen Träger 10, positioniert ist. Das Anschlussstück 11 selbst erstreckt sich vorzugsweise bis an die Rückseite des Trägers 10 der Anschlussvorrichtung 8 und umfasst dort vorzugsweise einen zweiten Kupplungsbereich 32. Der zweite Kupplungsbereich 32 dient zur Kupplung der Reinigungs- oder Pflegekassette 1, insbesondere deren Anschlussvorrichtung 8 mit den daran angeschlossenen zu reinigenden und/ oder pflegenden Instrumenten, mit einer Medienzuführung eines Reinigungs- oder Pflegegeräts zur Reinigung und/ oder Pflege der im Inneren der medizinischen Instrumente angeordneten Versorgungs- und Fluidleitungen.

In Figur 6 ist ein Ausführungsbeispiel der Anschlussvorrichtung 8 der Reinigungs- oder Pflegekassette 1 abgebildet. Die Anschlussvorrichtung 8 mit einem Träger 10, aufweisend eine erste und zweite Außenseite 14, 15 sowie eine Stirnseite 34, und zumindest einem Anschlussstück 11 ist drehbar um die Querachse 13 gelagert, wobei die Querachse 13 vorzugsweise gleich beabstandet zu den Seiten 14, 15 der Anschlussvorrichtung 8 angeordnet ist, welche den Deckel- oder Bodenflächen 4, 5 des ersten und zweiten Gehäuseteils 2, 3 zugewandt sind. In dem bevorzugten Ausführungsbeispiel ist die Anschlussvorrichtung 8, zumindest der Träger 10 der Anschlussvorrichtung 8, drehsymmetrisch zur Drehsymmetrieachse 31 der Reinigungs-oder Pflegekassette 1 ausgebildet. Hierdurch wird es ermöglicht, dass die mittels der Anschlussvorrichtung in der Reinigungs- oder Pflegekassette aufgenommen medizinischen Instrumente bei Öffnen des ersten und/ oder zweiten Gehäuseteils 2, 3 gleich zugänglich sind.

Die Erfindung ist nicht auf das beschriebene Ausführungsbeispiel beschränkt, sondern umfasst alle Ausführungen, die das prinzipielle, sinngemäße Funktionsprinzip der Erfindung anwenden oder beinhalten.

## Patentansprüche

1. Reinigungs- oder Pflegekassette (1) zur Aufnahme von medizinischen, insbesondere zahnärztlichen, Instrumenten, aufweisend ein Gehäuse mit zumindest einem ersten und zweiten Gehäuseteil (2, 3), welche jeweils zumindest eine Deckel- oder Bodenfläche (4, 5) sowie zumindest eine Seitenfläche (6, 7) aufweisen, und zumindest eine zwischen den zwei Gehäuseteilen (2, 3) angeordnete Anschlussvorrichtung (8) für zumindest ein medizinisches, insbesondere zahnärztliches, Instrument, wobei das erste und zweite Gehäuseteil (2, 3) drehbar über eine Drehachse (9) miteinander verbunden sind, **dadurch gekennzeichnet, dass** die Drehachse (9) gleich beabstandet zu den Deckel- oder Bodenflächen (4, 5) beider Gehäuseteile (2, 3) angeordnet ist.

2. Reinigungs- oder Pflegekassette (1) nach Anspruch 1, **dadurch gekennzeichnet, dass** die Anschlussvorrichtung (8) für das zumindest eine medizinische, insbesondere zahnärztliche, Instrument die Drehachse (9) der Reinigungs-oder Pflegekassette (1) bildet, um welche zumindest ein Gehäuseteil (2, 3) drehbar ist.

3. Reinigungs- oder Pflegekassette (1) nach Anspruch 1 oder 2, **dadurch gekennzeichnet, dass** die Anschlussvorrichtung (8) einen Träger (10) und zumindest ein Anschlussstück (11) aufweist, um ein medizinisches, insbesondere zahnärztliches Instrument, oder einen Anschlusszapfen (12) für ein medizinisches, insbesondere zahnärztliches, Instrument aufzunehmen.

4. Reinigungs- oder Pflegekassette (1) nach Anspruch 3, **dadurch gekennzeichnet, dass** das zumindest eine Anschlussstück (11) der Anschlussvorrichtung (8) relativ zu dem Träger (10) drehbar gelagert ist, so dass das mit dem Anschlussstück (11) verbundene medizinische, insbesondere zahnärztliche, Instrument relativ zu dem Träger (10) drehbar ist.

5. Reinigungs- oder Pflegekassette (1) nach einem der vorherigen Ansprüche, **dadurch gekennzeichnet, dass** die Anschlussvorrichtung (8) drehbar um eine Querachse (13) gelagert ist, wobei bevorzugt die Querachse (13) gleich beabstandet zu den Seiten (14, 15) der Anschlussvorrichtung (8) angeordnet ist, welche den Deckel- oder Bodenflächen (4, 5) des ersten und zweiten Gehäuseteils (2, 3) zugewandt sind.

6. Reinigungs- oder Pflegekassette (1) nach einem der vorherigen Ansprüche, **dadurch gekennzeichnet, dass** die Mittelachse (16) des zumindest einen Anschlussstücks (11) die Querachse (13) und/ oder die Drehachse (9) der Anschlussvorrichtung (8) schneidet, so dass das Anschlussstück (11) mittig an der Anschlussvorrichtung (8) positioniert ist.

7. Reinigungs- oder Pflegekassette (1) nach einem der vorherigen Ansprüche, **dadurch gekennzeichnet, dass** die Querachse (13) der Anschlussvorrichtung (8) in der Drehachse (9) der zumindest zwei Gehäuseteile (2, 3) der Reinigungs-oder Pflegekassette (1) liegt.

8. Reinigungs- oder Pflegekassette (1) nach einem der vorherigen Ansprüche, **dadurch gekennzeichnet, dass** im geschlossenen Zustand der Reinigungs-oder Pflegekassette (1) die Kontaktflächen (17) des ersten und zweiten Gehäuseteils (2, 3), an denen die beiden Seitenflächen (6, 7) des ersten Gehäuseteils (2, 3) und des zweiten Gehäuseteils (2, 3) einander kontaktieren, die Drehachse (9) der Reinigungs- oder Pflegekassette (1) schneiden.

9. Reinigungs- oder Pflegekassette (1) nach einem der vorherigen Ansprüche, **dadurch gekennzeichnet, dass** das erste und zweite Gehäuseteil (2, 3) jeweils zumindest zwei Lagerstellen (18, 19; 20, 21) zur Aufnahme der Drehachse (9) der Reinigungs- oder Pflegekassette (1) aufweist, wobei in montiertem Zustand eine erste Lagerstelle (19) des ersten Gehäuseteils (2) zwischen den zwei Lagerstellen (20, 21) des zweiten Gehäuseteils (3) und die zweite Lagerstelle (18) außerhalb angeordnet ist.

10. Reinigungs- oder Pflegekassette (1) nach einem der vorherigen Ansprüche, **dadurch gekennzeichnet, dass** das zumindest eine erste und zweite Gehäuseteil (2, 3) jeweils zumindest ein Verriegelungselement (22, 23) aufweist, so dass die zumindest zwei Gehäuseteile (2, 3) der Reinigungs- oder Pflegekassette (1) im geschlossenen Zustand miteinander lösbar verriegelbar sind.

11. Reinigungs- oder Pflegekassette (1) nach einem der vorherigen Ansprüche, **dadurch gekennzeichnet, dass** das erste und zweite Gehäuseteil (2, 3) jeweils zumindest eine Öffnung (24, 25) zur Aufnahme zumindest einer Reinigungsdüse eines Reinigungs- oder Pflegegeräts in der Reinigungs- oder Pflegekassette (1) aufweist, wobei die zumindest eine Öffnung (24, 25) bevorzugt an der der Anschlussvorrichtung (8) gegenüberliegenden Seite der Reinigungs- oder Pflegekassette (1) angeordnet ist.

12. Reinigungs- oder Pflegekassette (1) nach einem der vorherigen Ansprüche, **dadurch gekennzeichnet, dass** das erste und/ oder zweite Gehäuseteil (2, 3) an der Außenseite der Deckel- oder Bodenfläche (4, 5) zumindest einen Gehäusevorsprung (26, 27) aufweist, so dass bei Anordnung der Reinigungs-oder Pflegekassette (1) in einem Reinigungs- oder Pflegegerät ein Reinigungs-oder Pflegemittel an der Außenseite der Reinigungs- oder Pflegekassette (1) vorbeileitbar ist.

13. Reinigungs- oder Pflegekassette (1) nach einem der vorherigen Ansprüche, **dadurch gekennzeichnet, dass** das erste und/ oder zweite Gehäuseteil (2, 3) an der Außenseite der Deckel- oder Bodenfläche (4, 5) zumindest einen Gehäuserücksprung (28) aufweist, so dass ein Gehäusevorsprung (26, 27) einer ersten Reinigungs- oder Pflegekassette (1) in einen Gehäuserücksprung (28) einer zweiten Reinigungs- oder Pflegekassette (1) aufnehmbar ist.

14. Reinigungs- oder Pflegekassette (1) nach einem der vorherigen Ansprüche, **dadurch gekennzeichnet, dass** das Gehäuse der Reinigungs- oder Pflegekassette (1) drehsymmetrisch ausgebildet ist.

15. Reinigungs- oder Pflegekassette (1) nach einem der vorherigen Ansprüche, **dadurch gekennzeichnet, dass** die Anschlussvorrichtung (8) der Reinigungs-oder Pflegekassette (1), insbesondere der Träger (10) der Anschlussvorrichtung, (8) drehsymmetrisch ausgebildet ist.
